# EUROPEAN PATENT APPLICATION

(11) **EP 4 825 326 A1**
(43) Date of publication of application: **21.10.2026**
(21) Application number: 24903406.7
(22) Date of filing: 12.11.2024
(51) Int. Cl.: A61B 17/12

(54) **INDWELLING SYSTEM FOR IN VIVO INDWELLING TOOL AND METHOD FOR MANUFACTURING IN VIVO INDWELLING TOOL**

(30) Priority: 11.12.2023 JP 2023208509
(71) Applicant: KANEKA CORPORATION, Osaka 530-8288 (JP)
(72) Inventor: NAKANISHI, Hidekazu, Settsu-shi, Osaka 566-0072 (JP)
(74) Representative: Plasseraud IP
(86) International application number: PCT/JP2024/040086
(87) International publication number: WO 2025/126746

(57) **Abstract**

An indwelling system (1) for an in-vivo indwelling instrument includes a first coil (11) and a second coil (21) having a coil hardness less than a hardness of the first coil (11), wherein a drug is arranged on a surface of the first coil (11), and a drug is arranged on a surface of the second coil (21) such that an amount of the drug per coil unit length on the second coil (21) is less than that on the first coil (11), or no drug is arranged on the surface of the second coil (21).

## Description

### TECHNICAL FIELD

The present invention relates to an indwelling system for an in-vivo indwelling instrument for embolization at a vascular disease site in a blood vessel, and a method for manufacturing the in-vivo indwelling instrument.

### BACKGROUND ART

Endovascular treatment is an example of treatment methods for vascular lesions such as aneurysms in the head and neck, arteriovenous malformations, arteriovenous fistulas, pulmonary vascular malformations, renovascular malformations, and renal artery and abdominal aneurysms. The endovascular treatment uses embolization in which an in-vivo indwelling instrument having a coil for embolization is indwelt at a target site, and thrombogenesis is thus promoted, so that, for example, rupture of the aneurysm is prevented. In the embolization, a procedure for filling the aneurysm with the coil is performed, and the procedure includes Framing, Filling, and Finishing phases. In the embolization, a coil is selected from coils having different flexibilities for each phase in general. For example, in the Framing phase, by arranging a coil along the inner surface of the aneurysm, a shape as a framework needs to be formed in the aneurysm. Meanwhile, in the Filling and the subsequent phases, a coil which is more flexible than that in the Framing is selected in order to fill the coil into the framework formed in the Framing. For performing the embolization once, several to several tens of coils are used. Patent Literatures 1 to 4 disclose an in-vivo indwelling instrument in which a drug is held by the coil.

### RELATED ART DOCUMENT

### PATENT LITERATURE

Patent Literature 1: US 2007/0299461 A1
Patent Literature 2: JP 2005-513081 A
Patent Literature 3: JP 2013-537046 A
Patent Literature 4: JP 2015-195978 A

### SUMMARY OF THE INVENTION

### PROBLEMS TO BE SOLVED BY THE INVENTION

Although all of the plurality of coils used for performing the procedure once may have drugs arranged thereon, the coil having the drug arranged thereon becomes hard, so that operability may be degraded. Therefore, an object of the present invention is to provide an indwelling system for an in-vivo indwelling instrument capable of applying a drug to an aneurysm at an amount required for treatment, and reducing influence of degradation of operability, and a method for manufacturing the in-vivo indwelling instrument.

### MEANS FOR SOLVING THE PROBLEMS

An indwelling system for an in-vivo indwelling instrument according to an embodiment of the present invention that can solve the above problems is as follows.
[1] An indwelling system for an in-vivo indwelling instrument, comprising:
   a first coil; and
   a second coil having a coil hardness less than a hardness of the first coil, wherein
   a drug is arranged on a surface of the first coil, and
   a drug is arranged on a surface of the second coil such that an amount of the drug per coil unit length on the second coil is less than that on the first coil, or no drug is arranged on the surface of the second coil.
      Furthermore, the indwelling system for the in-vivo indwelling instrument according to the embodiment is preferably any one of the following [2] to [10].
[2] The indwelling system for the in-vivo indwelling instrument according to [1], wherein the drug is arranged on the first coil over an entirety of the first coil in a longitudinal axis direction.
[3] The indwelling system for the in-vivo indwelling instrument according to [1] or [2], wherein the drug is arranged on the first coil on an outer circumferential surface and an inner circumferential surface of the first coil.
[4] The indwelling system for the in-vivo indwelling instrument according to any one of [1] to [3], wherein the drug is arranged on the first coil on an outer circumferential surface of the first coil and is not arranged on an inner circumferential surface of the first coil.
[5] The indwelling system for the in-vivo indwelling instrument according to any one of [1] to [4], further comprising a first stretch resistant member disposed in a lumen of the first coil, wherein
   a drug is arranged on a surface of the first stretch resistant member.
[6] The indwelling system for the in-vivo indwelling instrument according to any one of [1] to [5], wherein the first coil has a coil hardness of 5.0×10⁻⁹ N/mm or more.
[7] The indwelling system for the in-vivo indwelling instrument according to any one of [1] to [6], further comprising a third coil having a coil hardness more than the hardness of the first coil, wherein
   a drug is arranged on a surface of the third coil such that an amount of the drug per coil unit length on the third coil is more than that on the first coil.
[8] The indwelling system for the in-vivo indwelling instrument according to [7], wherein the third coil has a coil hardness of 3.5×10⁻⁸ N/mm or more.
[9] The indwelling system for the in-vivo indwelling instrument according to any one of [1] to [8], wherein at least one of the drug arranged on the first coil and the drug arranged on the second coil is encapsulated in a capsule containing a biodegradable material.
[10] The indwelling system for the in-vivo indwelling instrument according to any one of [1] to [9], wherein at least one of the drug arranged on the first coil and the drug arranged on the second coil has at least one of an anti-inflammatory effect, an antioxidant effect, an antihypertensive effect, a vasoconstriction inhibitory effect, an anticoagulant effect, and a shear stress sensing inhibitory effect.
   A method for manufacturing the in-vivo indwelling instrument according to an embodiment of the present invention that can solve the above problems is as follows.
[11] A method for manufacturing an in-vivo indwelling instrument, the method comprising applying a drug when a coil has a hardness of a first reference value or more, and applying no drug when the coil has a hardness of less than the first reference value.
   Furthermore, the method for manufacturing the in-vivo indwelling instrument according to the embodiment is preferably one of the following [12] to [14].
[12] The method for manufacturing the in-vivo indwelling instrument according to [11], wherein the first reference value is 5.0×10⁻⁹ N/mm or more and 1.5×10⁻⁸ N/mm or less.
[13] The method for manufacturing the in-vivo indwelling instrument according to [11] or [12], wherein, in applying the drug when the coil has a hardness of the first reference value or more and applying no drug when the coil has a hardness of less than the first reference value, when the hardness of the coil is more than or equal to a second reference value more than the first reference value, an amount of the drug to be applied per coil unit length is made more than that in a case where the hardness of the coil is the first reference value or more and less than the second reference value.
[14] The method for manufacturing the in-vivo indwelling instrument according to [13], wherein the second reference value is 3.5×10⁻⁸ N/mm or more and 4.5×10⁻⁸ N/mm or less.

### ADVANTAGEOUS EFFECTS OF THE INVENTION

The indwelling system for the in-vivo indwelling instrument allows the coils to be indwelt in an aneurysm while ensuring operability since an amount of the drug arranged on the second coil which is more flexible than the first coil is less than that on the first coil, or no drug is arranged on the second coil, and the second coil thus maintains flexibility. Since the first coil is harder than the second coil, the rate of change in coil hardness between before and after application of the drug is less on the first coil than on the second coil. Therefore, even when the coil becomes hard due to application of the drug to the first coil, influence of degradation of operability can be reduced on the first coil. The indwelling system for the in-vivo indwelling instrument which can apply a drug to an affected site at an amount required for treatment, and can also contribute to ensuring operability can be provided by combining the first coil and the second coil described above.

In the method for manufacturing the in-vivo indwelling instrument, the drug is applied to the coil in which a rate of change in coil hardness between before and after application of the drug is small, and therefore, even when the coil becomes hard due to application of the drug, influence of degradation of operability of the coil can be reduced. The coil having such a configuration also allows the drug to be applied to an affected site at an amount required for treatment.

### BRIEF DESCRIPTION OF THE DRAWINGS

[FIG. 1] FIG. 1 is a schematic diagram illustrating the indwelling system for the in-vivo indwelling instrument according to one embodiment of the present invention.
[FIG. 2] FIG. 2 is a cross-sectional view (a part thereof is a side view) of the first coil shown in FIG. 1 as taken along the longitudinal axis direction.
[FIG. 3] FIG. 3 is a cross-sectional view (a part thereof is a side view) of a modification of the first coil shown in FIG. 2.
[FIG. 4] FIG. 4 is a cross-sectional view (a part thereof is a side view) of the second coil shown in FIG. 1 as taken along the longitudinal axis direction.
[FIG. 5] FIG. 5 is a cross-sectional view (a part thereof is a side view) of a modification of the second coil shown in FIG. 4.
[FIG. 6] FIG. 6 is a cross-sectional view (a part thereof is a side view) of another modification of the second coil shown in FIG. 4.
[FIG. 7] FIG. 7 is a schematic diagram illustrating a modification of the indwelling system for the in-vivo indwelling instrument as shown in FIG. 1.
[FIG. 8] FIG. 8 is a cross-sectional view (a part thereof is a side view) of the third coil shown in FIG. 7 as taken along the longitudinal axis direction.
[FIG. 9] FIG. 9 is a cross-sectional view (a part thereof is a side view) of a modification of the third coil shown in FIG. 8.
[FIG. 10] FIG. 10 is a cross-sectional view of the first stretch resistant member shown in FIG. 2 as taken along the direction perpendicular to the longitudinal axis direction.
[FIG. 11] FIG. 11 is a cross-sectional view of a modification of the first stretch resistant member shown in FIG. 10.
[FIG. 12] FIG. 12 is a cross-sectional view of the second stretch resistant member shown in FIG. 4 as taken along the direction perpendicular to the longitudinal axis direction.
[FIG. 13] FIG. 13 is a cross-sectional view of a modification of the second stretch resistant member shown in FIG. 12.
[FIG. 14] FIG. 14 is a cross-sectional view of the third stretch resistant member shown in FIG. 8 as taken along the direction perpendicular to the longitudinal axis direction.
[FIG. 15] FIG. 15 is a cross-sectional view of a modification of the third stretch resistant member shown in FIG. 14.
[FIG. 16] FIG. 16 is a flow chart showing a method for manufacturing the in-vivo indwelling instrument according to one embodiment of the present invention.
[FIG. 17] FIG. 17 is a flow chart showing a modification of the method for manufacturing the in-vivo indwelling instrument as shown in FIG. 16.

### EMBODIMENTS FOR CARRYING OUT THE INVENTION

The present invention will be specifically explained below based on the following embodiments, however, the present invention is not restricted by the embodiments described below of course, and can be certainly put into practice after appropriate modifications within in a range meeting the gist of the above and the below, all of which are included in the technical scope of the present invention. In the drawings, hatching, a reference sign for a member may be omitted for convenience, and in such a case, the description and other drawings should be referred to. In addition, sizes of various members in the drawings may differ from the actual sizes thereof, since priority is given to understanding the features of the present invention.

### 1. Indwelling system for in-vivo indwelling instrument

Hereinafter, an indwelling system for an in-vivo indwelling instrument may be simply referred to as indwelling system, and an in-vivo indwelling instrument may be simply referred to as indwelling instrument. Examples of the application of the indwelling system include embolizations for promoting thrombogenesis at target sites such as cerebral aneurysms, aneurysms in the head and neck, arteriovenous malformations, arteriovenous fistulas, pulmonary vascular malformations, renovascular malformations, and renal artery and abdominal aneurysms. Among them, the indwelling system is preferably an indwelling system for cerebral aneurysms. Examples of the shape of the aneurysm include a fusiform shape and a saccular shape.

The present invention has a gist that the indwelling system according to one embodiment of the present invention has a first coil and a second coil having a coil hardness less than a hardness of the first coil, a drug is arranged on a surface of the first coil, and a drug is arranged on a surface of the second coil such that an amount of the drug per coil unit length on the second coil is less than that on the first coil, or no drug is arranged on the surface of the second coil. The above-described indwelling system for the in-vivo indwelling instrument allows the coils to be indwelt in an aneurysm while ensuring operability since an amount of the drug arranged on the second coil which is more flexible than the first coil is less than that on the first coil, or no drug is arranged on the second coil, and the second coil thus maintains flexibility. Since the first coil is harder than the second coil, the rate of change in coil hardness between before and after application of the drug is less on the first coil than on the second coil. Therefore, even when the coil becomes hard due to application of the drug to the first coil, influence of degradation of operability can be reduced on the first coil. The indwelling system for the in-vivo indwelling instrument which can apply a drug to an affected site at an amount required for treatment, and can also contribute to ensuring operability can be provided by combining the first coil and the second coil described above.

The indwelling system preferably has a first in-vivo indwelling instrument including the first coil, and a second in-vivo indwelling instrument including the second coil. The first in-vivo indwelling instrument preferably has the first coil and a first stretch resistant member disposed in a lumen of the first coil. The second in-vivo indwelling instrument preferably has the second coil and a second stretch resistant member disposed in a lumen of the second coil. The indwelling system preferably has a plurality of coils including the first coil, the second coil, and a third coil. The indwelling system preferably has the first in-vivo indwelling instrument including the first coil, the second in-vivo indwelling instrument including the second coil, and a third in-vivo indwelling instrument including the third coil. The third in-vivo indwelling instrument preferably has the third coil, and a third stretch resistant member disposed in a lumen of the third coil.

The embolization includes Framing, Filling, and Finishing phases. Each of the first coil and the second coil of the indwelling system can be used for any one of the phases, or used over two or three of the phases. Particularly, the first coil is preferably used for the Framing and/or the Filling. The second coil is preferably used for the Finishing. The third coil described below is preferably used for the Framing and/or the Filling, and more preferably used for the Framing.

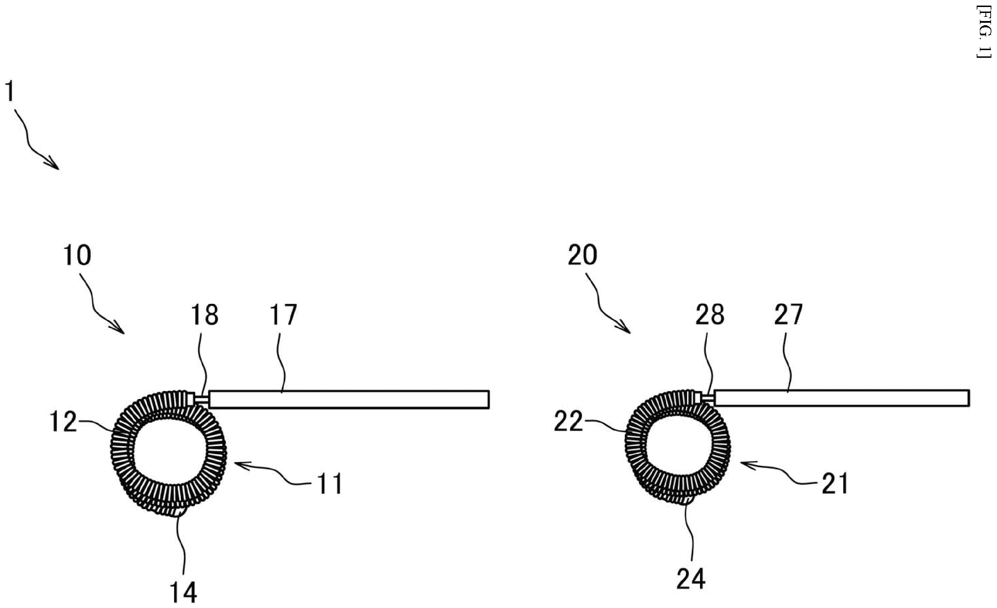

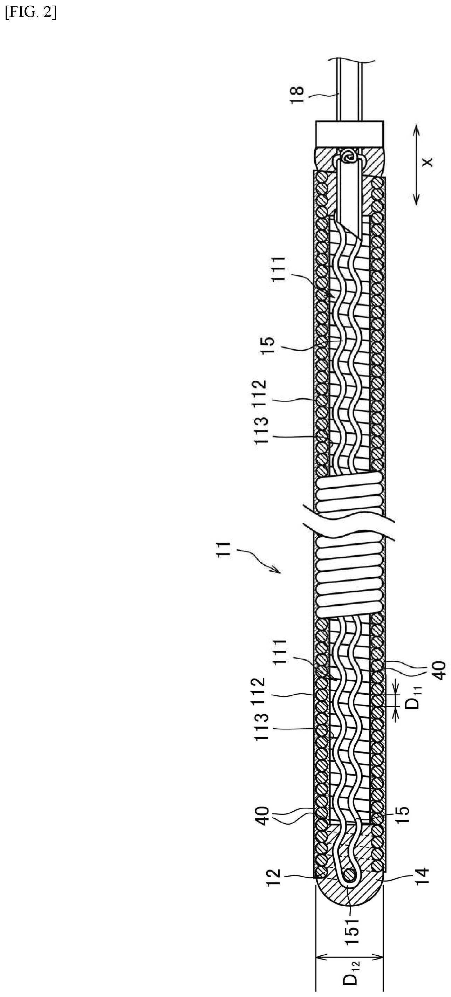

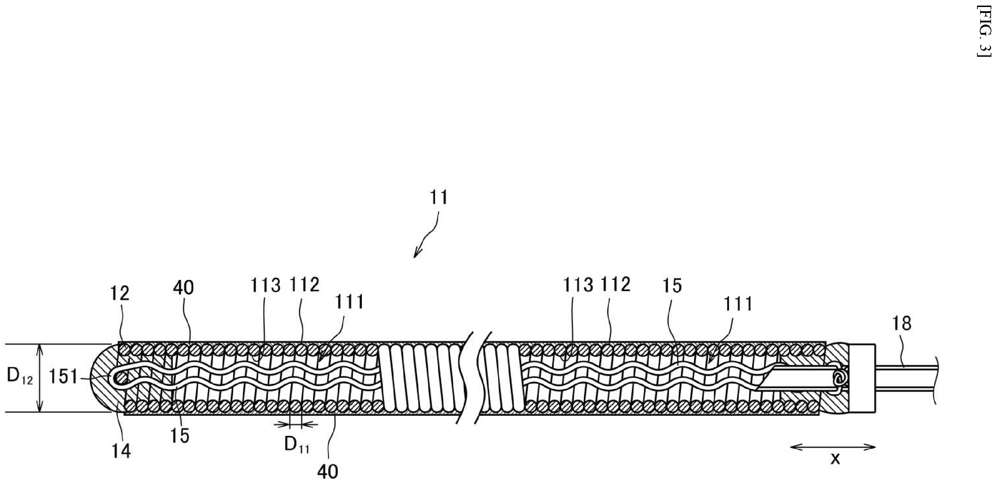

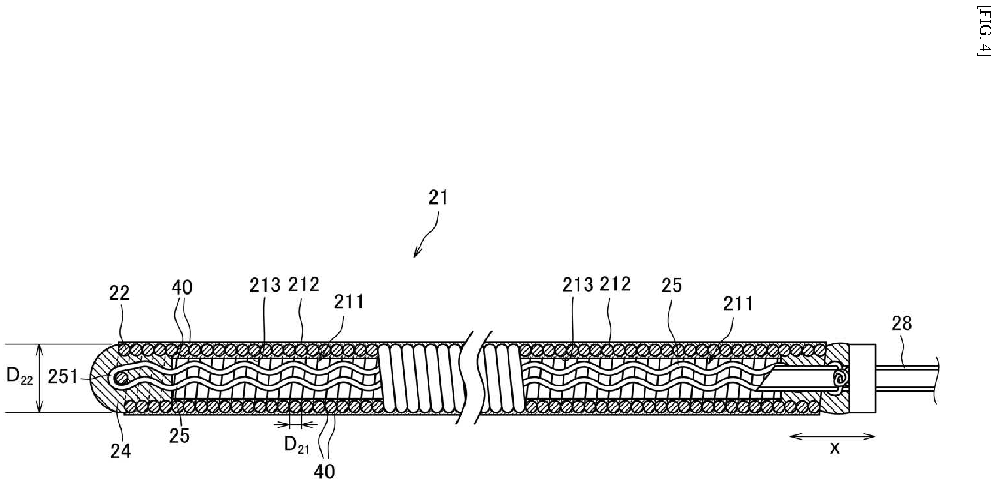

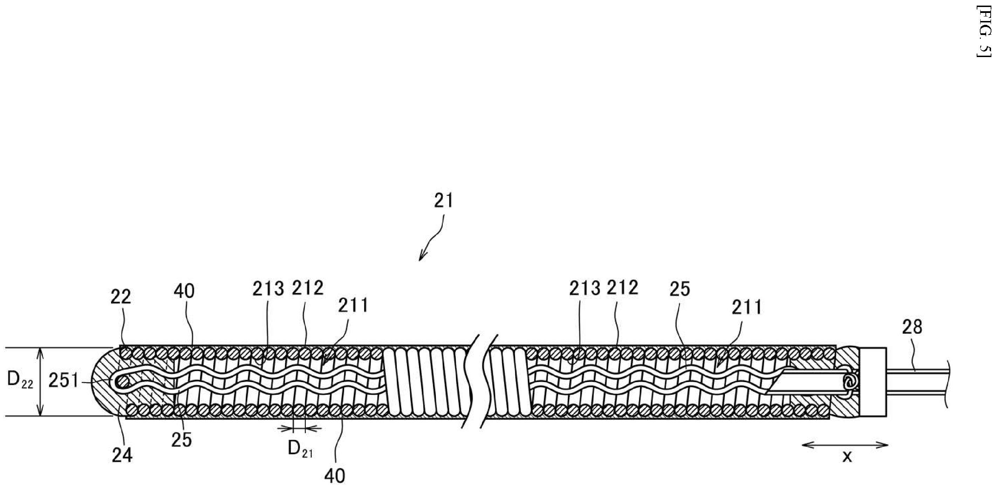

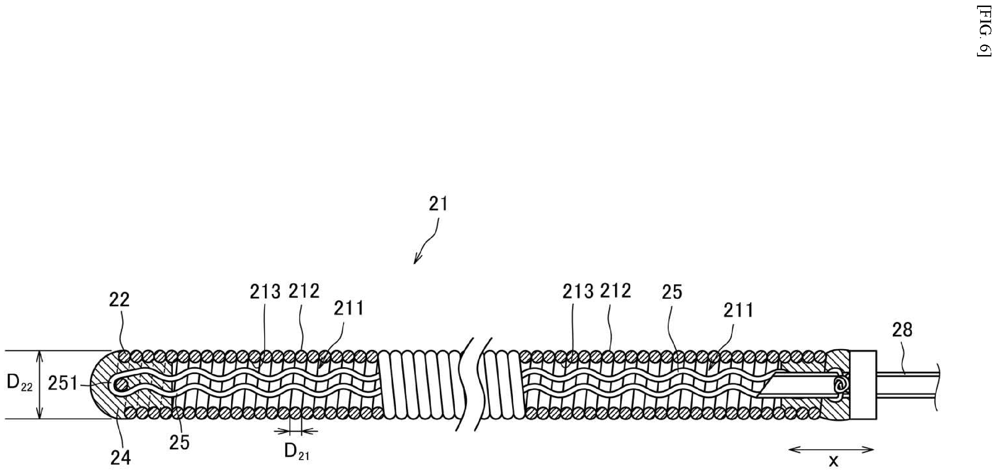

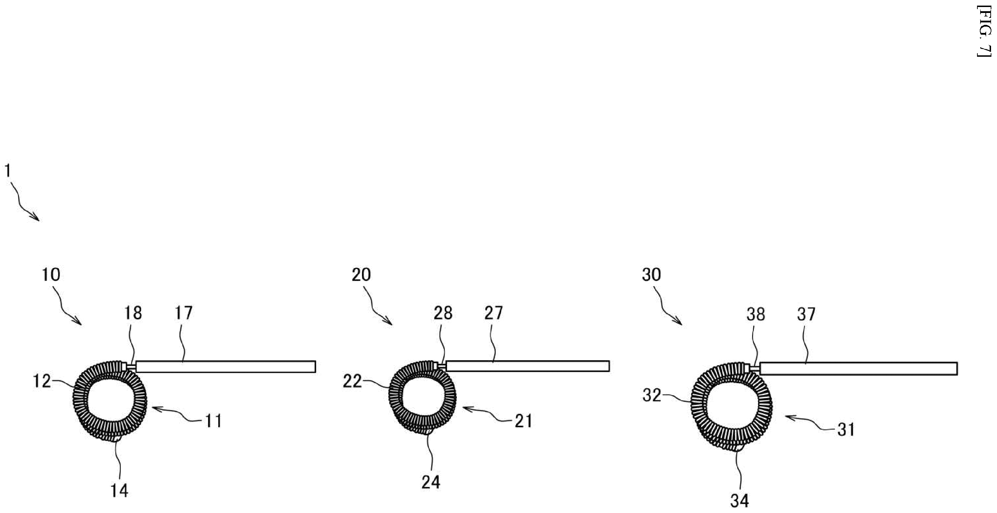

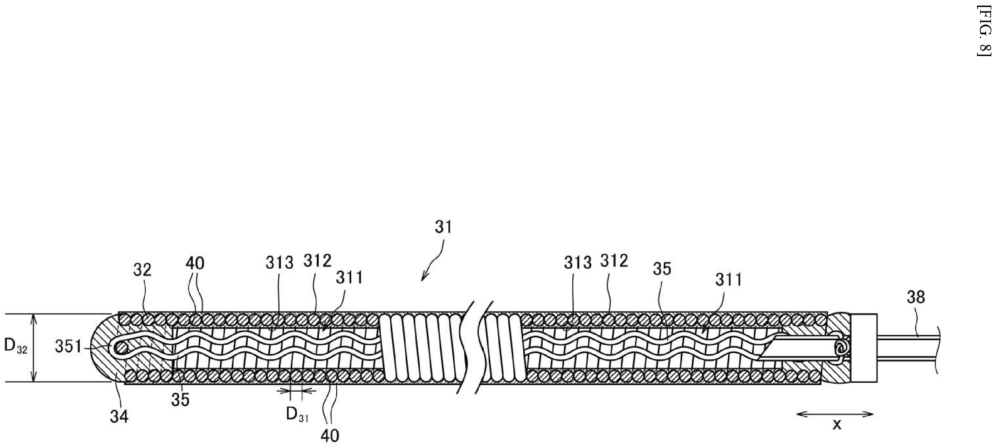

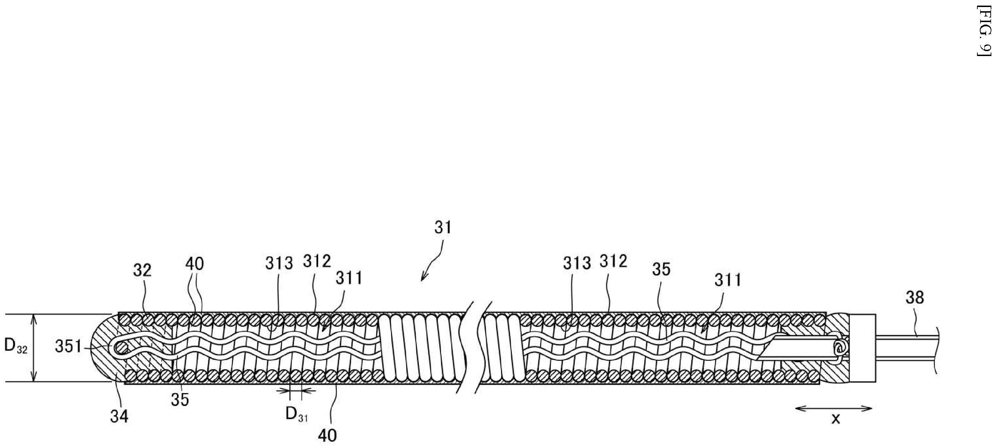

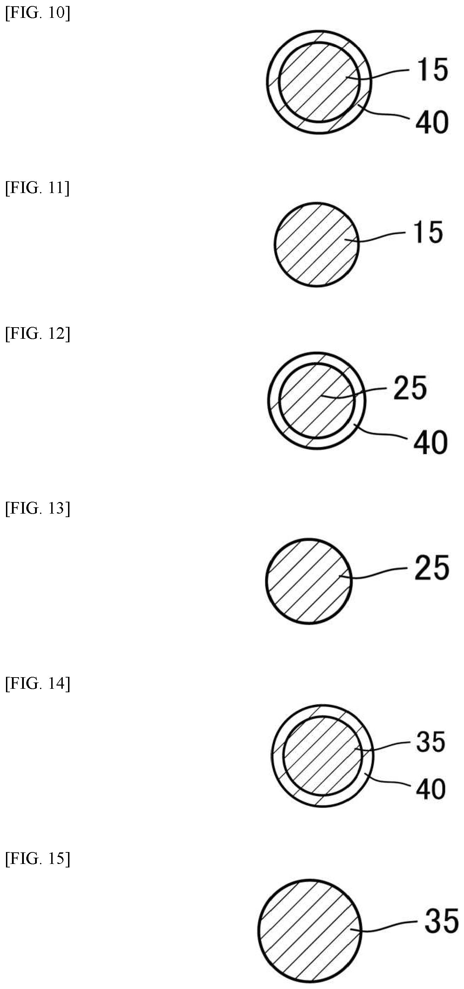

With reference to FIG. 1 to FIG. 15, the indwelling system for the in-vivo indwelling instrument according to one embodiment of the present invention will be described. FIG. 1 is a schematic diagram illustrating the indwelling system for the in-vivo indwelling instrument according to one embodiment of the present invention. FIG. 2 is a cross-sectional view (a part thereof is a side view) of the first coil shown in FIG. 1 as taken along the longitudinal axis direction. FIG. 3 is a cross-sectional view (a part thereof is a side view) of a modification of the first coil shown in FIG. 2. FIG. 4 is a cross-sectional view (a part thereof is a side view) of the second coil shown in FIG. 1 as taken along the longitudinal axis direction. FIG. 5 is a cross-sectional view (a part thereof is a side view) of a modification of the second coil shown in FIG. 4. FIG. 6 is a cross-sectional view (a part thereof is a side view) of another modification of the second coil shown in FIG. 4. FIG. 7 is a schematic diagram illustrating a modification of the indwelling system for the in-vivo indwelling instrument as shown in FIG. 1. FIG. 8 is a cross-sectional view (a part thereof is a side view) of the third coil shown in FIG. 7 as taken along the longitudinal axis direction. FIG. 9 is a cross-sectional view (a part thereof is a side view) of a modification of the third coil shown in FIG. 8. FIG. 10 is a cross-sectional view of the first stretch resistant member shown in FIG. 2 as taken along the direction perpendicular to the longitudinal axis direction. FIG. 11 is a cross-sectional view of a modification of the first stretch resistant member shown in FIG. 10. FIG. 12 is a cross-sectional view of the second stretch resistant member shown in FIG. 4 as taken along the direction perpendicular to the longitudinal axis direction. FIG. 13 is a cross-sectional view of a modification of the second stretch resistant member shown in FIG. 12. FIG. 14 is a cross-sectional view of the third stretch resistant member shown in FIG. 8 as taken along the direction perpendicular to the longitudinal axis direction. FIG. 15 is a cross-sectional view of a modification of the third stretch resistant member shown in FIG. 14. As shown in FIG. 1, an indwelling system 1 has a first coil 11 and a second coil 21.

Hereinafter, when a structure common to the first coil 11 and the second coil 21 is described, these coils or each coil may be simply referred to as coil.

As can be understood from FIG. 2 to FIG. 6, the coil preferably has a longitudinal axis direction x, a circumferential direction, and a radial direction. The coil preferably has a distal end and a proximal end in the longitudinal axis direction x. The proximal side of the coil refers to a side closer to a hand of a user or an operator with respect to the longitudinal axis direction x of the coil, and the distal side refers to a side opposite to the proximal side, i.e., a side closer to a target to be treated. In FIG. 2 to FIG. 6 and FIG. 8 to FIG. 9, the right side in the figures is the proximal side, and the left side in the figures is the distal side. The radial direction of the coil refers to a coil radius direction. The inner direction in the radial direction of the coil refers to a direction toward the longitudinal axis center side of the coil. The outer direction in the radial direction refers to a direction radially extending from the longitudinal axis center toward the side opposite to the inner direction side. The circumferential direction of the coil refers to a direction around the longitudinal axis.

The first coil 11 and the second coil 21 may have the same length or may have lengths different from each other in the longitudinal axis direction x. The second coil 21 may be longer than the first coil 11 in the longitudinal axis direction x. In the longitudinal axis direction x, the first coil 11 may be longer than the second coil 21.

As shown in FIG. 1 to FIG. 3, the first coil 11 is preferably composed of a first wire 12. As shown in FIG. 1 and FIG. 4 to FIG. 6, the second coil 21 is preferably composed of a second wire 22. Hereinafter, when a structure common to the first wire 12 and the second wire 22 is described, these wires or each wire may be simply referred to as wire.

The coil has a structure in which one wire or a plurality of wires are helically wound. Examples of the wire include a single wire, a stranded wire, and a coil wire wound into a coil-like shape. Among them, a single wire is preferable. The wire is preferably other than a coil wire. Each of the first wire 12 and the second wire 22 is preferably a single wire.

A coil formed by helically winding the wire may be referred to as primary coil. A coil formed by further bending and forming the primary coil into a helical shape or a three-dimensional shape may be referred to as secondary coil. In the description herein, unless otherwise specified, the coil refers to a coil structured as the primary coil. The primary coil as shown in FIG. 2 to FIG. 6 and FIG. 8 to FIG. 9 is preferably bent and formed into the secondary coil as shown in FIG. 1 and FIG. 7.

The wire preferably has biocompatibility and flexibility. Examples of a material forming the wire include metal materials such as platinum, gold, titanium, tungsten, alloys thereof, and stainless steel, and combinations thereof. Among them, the wire is more preferably formed of a platinum-tungsten alloy.

The wire has a longitudinal axis direction, and has a distal end and a proximal end in the longitudinal axis direction of the wire. The wire may be composed of a single wire member from the distal end to the proximal end, or may be composed of a plurality of wire members connected to each other in the longitudinal axis direction. The shape of the cross-section of the wire, which is perpendicular to the longitudinal axis direction, may be a circular, an oblong, or a polygonal shape, or a combination thereof. The shape of the cross-section of the wire, which is perpendicular to the longitudinal axis direction, may be the same over the entirety of the wire in the longitudinal axis direction, or may be different depending on the position in the longitudinal axis direction.

The outer diameter of the wire is not particularly limited, and may be, for example, 25 µm or more, 30 µm or more, or 35 µm or more, and is allowed to be 75 µm or less or 70 µm or less.

An outer diameter D₁₁ of the first wire 12 and an outer diameter D₂₁ of the second wire 22 may be equal to each other. The outer diameter D₁₁ of the first wire 12 and the outer diameter D₂₁ of the second wire 22 may be different from each other. The outer diameter D₁₁ of the first wire 12 may be more than the outer diameter D₂₁ of the second wire 22 such that the first coil 11 is likely to become harder than the second coil 21. The outer diameter D₁₁ of the first wire 12 may be less than the outer diameter D₂₁ of the second wire 22.

The outer diameter of the wire may be the same in the longitudinal axis direction of the wire or may be different depending on the position in the longitudinal axis direction of the wire. In a case where the cross-section of the wire has a shape other than a circular shape, the outer diameter of the wire represents an equivalent circle diameter.

As shown in FIG. 2 to FIG. 3, the first coil 11 preferably has a lumen 111 extending in the longitudinal axis direction x. The first coil 11 preferably has an outer circumferential surface 112 and an inner circumferential surface 113. The outer circumferential surface 112 of the first coil 11 faces the outer side of the first coil 11, i.e., the outer side in the radial direction. The inner circumferential surface 113 of the first coil 11 faces the lumen 111. A first stretch resistant member 15 described below is preferably disposed in the lumen 111.

As shown in FIG. 4 to FIG. 6, the second coil 21 preferably has a lumen 211 extending in the longitudinal axis direction x. The second coil 21 preferably has an outer circumferential surface 212 and an inner circumferential surface 213. The outer circumferential surface 212 of the second coil 21 faces the outer side of the second coil 21, i.e., the outer side in the radial direction. The inner circumferential surface 213 of the second coil 21 faces the lumen 211. A second stretch resistant member 25 described below is preferably disposed in the lumen 211.

The coil may be a monolayer coil or a multilayer coil having a plurality of layers. A part of the coil in the longitudinal axis direction x may be a monolayer, and the remaining part may be a multilayer.

The density of the coil, i.e., a winding interval is not particularly limited, and tight winding, pitch winding, or a combination thereof may be used. The wires adjacent to each other at a part of the coil in the longitudinal axis direction x may be in contact with each other, or the wires adjacent to each other over the entirety of the coil in the longitudinal axis direction x may be in contact with each other. A state in which the wires adjacent to each other in the longitudinal axis direction x of the coil are in contact with each other is referred to as tight winding, and a state in which the adjacent wires are not in contact with each other is referred to as pitch winding. A state in which the adjacent wires are not in contact with each other refers to a state in which the wires adjacent to each other in the longitudinal axis direction x of the coil are distant from each other. Each of the first coil 11 and the second coil 21 is preferably a tight-winding type coil.

The shape of the cross-section of the coil, which is perpendicular to the longitudinal axis direction x, may be a circular, an oblong, or a polygonal shape, or a combination thereof. The oblong shape includes an ellipsoidal, an oval, and a corner-rounded rectangular shape. The same applies to the following description.

The maximum outer diameter and the minimum outer diameter of the coil are not particularly limited, and can be selected as appropriate according to the phase of the procedure. However, the outer diameter of the coil may be, for example, 150 µm or more, 180 µm or more, or 200 µm or more, and is allowed to be 400 µm or less, 380 µm or less, or 350 µm or less.

An outer diameter D₁₂ of the first coil 11 and an outer diameter D₂₂ of the second coil 21 may be equal to each other. The outer diameter D₁₂ of the first coil 11 and the outer diameter D₂₂ of the second coil 21 may be different from each other. The outer diameter D₁₂ of the first coil 11 may be less than the outer diameter D₂₂ of the second coil 21 such that the first coil 11 is likely to become harder than the second coil 21. The outer diameter D₁₂ of the first coil 11 may be more than the outer diameter D₂₂ of the second coil 21. The outer diameter D₁₂ of the first coil 11 and the outer diameter D₂₂ of the second coil 21 each represent an average value of the outer diameters in the longitudinal axis direction x of the coil.

The outer diameters and/or the inner diameters of the coil may be the same in the longitudinal axis direction x of the coil or may be different depending on the position in the longitudinal axis direction x of the coil. In a case where the cross-section of the coil has a shape other than a circular shape, the outer diameter of the coil represents an equivalent circle diameter. In a case where a lumen cross-section of the coil has a shape other than a circular shape, the inner diameter of the coil represents an equivalent circle diameter.

The coil may have a constant outer diameter in the longitudinal axis direction x. The constant outer diameter means that the outer diameter of the coil is substantially constant over the entirety of the coil in the longitudinal axis direction x, and also means that the change in the outer diameter of the coil over the entirety thereof in the longitudinal axis direction x is within a range of ±5%.

The coil may have a transition portion in which the outer diameter becomes smaller toward the distal side, which is not shown. Thus, flexibility of the coil can be gradually enhanced from the distal side toward the proximal side. In the transition portion, the outer diameter of the coil may be reduced toward the distal side in a tapered state. In the description herein, the tapered state includes a state in which the outer diameter is reduced each time the wire forming the coil is wound once, so that the envelope of the outer diameter of the coil is tapered at the transition portion. At the transition portion, the outer diameter of the coil may be reduced stepwise toward the distal side. In the description herein, the stepwise state includes a state in which the outer diameter is reduced each time the wire forming the coil is wound at least twice.

When the coil is equally divided into two portions which are a distal portion and a proximal portion in the longitudinal axis direction x, the average value of the outer diameters at the proximal portion in the coil may be less than the average value of the outer diameters at the distal portion. In a case where the outer diameter is small at the proximal portion, the flexibility of the coil at the proximal portion can be enhanced, so that operability can be ensured.

In the description herein, the hardness of the coil refers to coil hardness in a state in which the coil is the primary coil. The coil hardness S₁ (unit: N/mm) of the first coil 11 (primary coil) and the coil hardness S₂ (unit: N/mm) of the second coil 21 (primary coil) can be calculated according to the following equations. ${S}_{1} = {{D}_{11}}^{4} \times G / \left(8{{D}_{12}}^{3}\times {n}_{1}\right)$ ${S}_{2} = {{D}_{21}}^{4} \times G / \left(8{{D}_{22}}^{3}\times {n}_{2}\right)$ in which D₁₁ represents the outer diameter (unit: mm) of the first wire 12, G represents a shear modulus (unit: Pa(N/mm²)), D₁₂ represents the outer diameter (unit: mm) of the first coil 11, n₁ represents the number of winding times in the first coil 11 (no unit), D₂₁ represents the outer diameter (unit: mm) of the second wire 22, D₂₂ represents the outer diameter (unit: mm) of the second coil 21, and n₂ represents the number of winding times in the second coil 21 (no unit). In a case where the outer diameter of the first wire 12 is not constant in the longitudinal axis direction of the first wire 12, the outer diameter D₁₁ of the first wire 12 represents the average value of the outer diameters in the longitudinal axis direction of the first wire 12. The same applies to the outer diameter D₂₁ of the second wire 22 and an outer diameter D₃₁ of a third wire 32 described below. In a case where the outer diameter of the first coil 11 is not constant in the longitudinal axis direction of the first coil 11, the outer diameter D₁₂ of the first coil 11 represents the average value of the outer diameters in the longitudinal axis direction of the first coil 11. The same applies to the outer diameter D₂₂ of the second coil 21 and an outer diameter D₃₂ of a third coil 31 described below. The number n₁ of winding times in the first coil 11 represents the number of winding times as counted when the first coil 11 is viewed from the side at such an angle that the number of winding times becomes maximum. The same applies to the number n₂ of winding times in the second coil 21 and the number n₃ of winding times in the third coil 31 described below. The shear modulus G indicates a value which is different for each material of the wire, and the shear modulus G indicates the same value when the chemical component of the constituent material of the wire is the same. The same applies to the following description. The hardness of the coil is preferably calculated before a drug is applied. For the coil in which a drug is applied to the surface or the coil subjected to coating, the hardness of the coil is measured after the drug and the coating are removed. The method for removing the drug and the coating is not limited, and, for example, a method in which the drug or the coating is dissolved in an organic solvent such as ethanol, methanol, and chloroform can be used.

When the coil hardness is calculated as described above, the values measured by the measuring means such as calipers, a micrometer, or an image dimension measuring device, may be used as the outer diameter D₁₁ of the first wire 12, the outer diameter D₁₂ of the first coil 11, the outer diameter D₂₁ of the second wire 22, and the outer diameter D₂₂ of the second coil 21. The same applies to the outer diameter D₃₁ of the third wire 32 described below, and the outer diameter D₃₂ of the third coil 31.

When the coil hardness is calculated as described above, a value indicated in a product catalogue for the coil may be used as at least one of the outer diameter D₁₁ of the first wire 12, the outer diameter D₁₂ of the first coil 11, the number n₁ of winding times in the first coil 11, the outer diameter D₂₁ of the second wire 22, the outer diameter D₂₂ of the second coil 21, and the number n₂ of winding times in the second coil 21. The same applies to the number n₃ of winding times in the third coil 31 described below.

The coil hardness of the first coil 11 is preferably 5.0×10⁻⁹ N/mm or more. Even when the first coil having such a hardness becomes hard due to application of a drug, influence of degradation of operability can be reduced.

The coil hardness of the first coil 11 is more preferably 8.0×10⁻⁹ N/mm or more and even more preferably 1.0×10⁻⁸ N/mm or more. The coil hardness of the first coil 11 is preferably less than 3.5×10⁻⁸ N/mm, and is allowed to be 3.0×10⁻⁸ N/mm or less or 2.0×10⁻⁸ N/mm or less. In a case where a drug is arranged on the coil having such a hardness, the drug can be applied to an affected site at an amount required for treatment while influence of degradation of operability is reduced.

The coil hardness of the second coil 21 is preferably less than 5.0×10⁻⁹ N/mm. The second coil having such a hardness maintains flexibility even when a drug is applied, and the coil can thus be indwelt in an aneurysm while operability is ensured. The coil hardness of the second coil 21 may be 1.0×10⁻⁹ N/mm or more or 2.0×10⁻⁹ N/mm or more, and is allowed to be 4.0×10⁻⁹ N/mm or less or 3.0×10⁻⁹ N/mm or less.

As can be understood from FIG. 7, the indwelling system 1 may further have the third coil 31 having the coil hardness which is more than that of the first coil 11. In a case where the indwelling system 1 has the third coil 31 in addition to the first coil 11 and the second coil 21, the coil can be properly selected and used according to the phase of the procedure, and options for the procedure can be increased.

As shown in FIG. 7 to FIG. 9, the third coil 31 is preferably composed of the third wire 32. The third coil 31 is preferably formed by winding the third wire 32. The third coil 31 preferably has a lumen 311 extending in the longitudinal axis direction x. The third coil 31 preferably has an outer circumferential surface 312 and an inner circumferential surface 313. The outer circumferential surface 312 of the third coil 31 faces the outer side of the third coil 31, and the inner circumferential surface 313 of the third coil 31 faces the lumen 311. A third stretch resistant member 35 described below is preferably disposed in the lumen 311.

The coil hardness S₃ (unit: N/mm) of the third coil 31 (primary coil) can be calculated according to the following equation. ${S}_{3} = {{D}_{31}}^{4} \times G / \left(8{{D}_{32}}^{3}\times {n}_{3}\right)$ in which D₃₁ represents the outer diameter (unit: mm) of the third wire 32, G represents a shear modulus (unit: Pa(N/mm²)), D₃₂ represents the outer diameter (unit: mm) of the third coil 31, and n₃ represents the number of winding times in the third coil 31 (no unit).

The first coil 11 and the third coil 31 may have the same length or may have lengths different from each other in the longitudinal axis direction x. The third coil 31 may be longer than the first coil 11 in the longitudinal axis direction x. The first coil 11 may be longer than the third coil 31 in the longitudinal axis direction x. The second coil 21 and the third coil 31 may have the same length or may have lengths different from each other in the longitudinal axis direction x. The third coil 31 may be longer than the second coil 21 in the longitudinal axis direction x. The second coil 21 may be longer than the third coil 31 in the longitudinal axis direction x.

The outer diameter D₃₁ of the third wire 32 and the outer diameter D₁₁ of the first wire 12 may be equal to each other. The outer diameter D₃₁ of the third wire 32 and the outer diameter D₁₁ of the first wire 12 may be different from each other. The outer diameter D₃₁ of the third wire 32 may be more than the outer diameter D₁₁ of the first wire 12 such that the third coil 31 is likely to become harder than the first coil 11. The outer diameter D₃₁ of the third wire 32 may be less than the outer diameter D₁₁ of the first wire 12.

The outer diameter D₃₁ of the third wire 32 and the outer diameter D₂₁ of the second wire 22 may be equal to each other. The outer diameter D₃₁ of the third wire 32 and the outer diameter D₂₁ of the second wire 22 may be different from each other. The outer diameter D₃₁ of the third wire 32 may be more than the outer diameter D₂₁ of the second wire 22 such that the third coil 31 is likely to become harder than the second coil 21. The outer diameter D₃₁ of the third wire 32 may be less than the outer diameter D₂₁ of the second wire 22.

The outer diameter D₁₂ of the first coil 11 and the outer diameter D₃₂ of the third coil 31 may be equal to each other. The outer diameter D₁₂ of the first coil 11 and the outer diameter D₃₂ of the third coil 31 may be different from each other. The outer diameter D₃₂ of the third coil 31 may be less than the outer diameter D₁₂ of the first coil 11 such that the third coil 31 is likely to become harder than the first coil 11. The outer diameter D₃₂ of the third coil 31 may be more than the outer diameter D₁₂ of the first coil 11.

The outer diameter D₂₂ of the second coil 11 and the outer diameter D₃₂ of the third coil 31 may be equal to each other. The outer diameter D₂₂ of the second coil 21 and the outer diameter D₃₂ of the third coil 31 may be different from each other. The outer diameter D₃₂ of the third coil 31 may be less than the outer diameter D₂₂ of the second coil 21 such that the third coil 31 is likely to become harder than the second coil 21. The outer diameter D₃₂ of the third coil 31 may be more than the outer diameter D₂₂ of the second coil 21.

The coil hardness of the third coil 31 is preferably 3.5×10⁻⁸ N/mm or more. Even when the third coil having such a hardness becomes hard due to application of a drug, influence of degradation of operability can be reduced.

The coil hardness of the third coil 31 may be 4.0×10⁻⁸ N/mm or more or 8.0×10⁻⁸ N/mm or more, and is allowed to be 3.0×10⁻⁷ N/mm or less, 2.0×10⁻⁷ N/mm or less, or 1.0×10⁻⁷ N/mm or less. In a case where a drug is arranged on the coil having such a hardness, the drug can be applied to an affected site at an amount required for treatment while influence of degradation of operability is reduced.

For the preferable configuration of the third coil 31 other than the above-described configurations, the above description for the configurations of the first coil 11 and the second coil 21 can be referred to.

In the procedure, the third coil 31, the first coil 11, and the second coil 21 are preferably inserted into a body in order, respectively. In the procedure, the first coil 11, the third coil 31, and the second coil 21 may be inserted into a body in order, respectively.

As shown in FIG. 2 to FIG. 3, in the indwelling system 1, a drug 40 is arranged on the surface of the first coil 11. As can be understood from FIG. 4 to FIG. 5, the drug 40 is arranged on the surface of the second coil 21 such that an amount (mg/mm) of the drug per coil unit length is less than that on the first coil 11, or no drug is arranged on the surface of the second coil 21 as shown in FIG. 6. Thus, the second coil 21 maintains flexibility, so that the coil can be indwelt in an aneurysm while operability is ensured.

The drug 40 may be formed of an active ingredient (drug substance) alone, or may be a mixture with another additive. Preferable examples of the additive include a base, a plasticizer, a stabilizer, and a surfactant. In the description herein, the amount of the drug represents a mass (mg) of the drug. In a case where the drug is a mixture containing the additive, the amount of the drug represents the mass of the mixture in the description herein.

The amount of the drug per unit length of the first coil 11 can be obtained by dividing the total amount (the total amount of the drug) of the drug 40 arranged on the surface of the first coil 11 by the length (entire length) of the first coil 11 in the longitudinal axis direction x. The amount of the drug per unit length of the second coil 21, and the amount of the drug per unit length of the third coil 31 described below can be obtained by the same method.

The amount of the drug (mg/mm²) per coil unit area on the surface of the second coil 21 may be less than the amount of the drug (mg/mm²) per coil unit area on the surface of the first coil 11. The amount of the drug per coil unit area on the surface of the first coil 11 can be obtained by dividing the total amount (the total amount of the drug) of the drug 40 arranged on the surface of the first coil 11 by the surface area of the first coil 11. The wires may adhere to each other in the first coil 11 in some cases. When the amount of the drug per unit area of the first coil 11 is calculated, the area in which the wires adhere to each other is not included in the surface area of the first coil 11. The amount of the drug per coil unit area on the surface of the second coil 21, and the amount of the drug per unit area on the surface of the third coil 31 described below can be obtained by the same method.

In a case where the amounts of the drugs arranged on different members (for example, the amounts of the drugs arranged on the surface of the first coil 11 and the surface of the second coil 21) are compared with each other, the amounts of the drugs are measured by the same method.

The amount of the drug (mg/mm) per coil unit length or the amount of the drug (mg/mm²) per coil unit area on each of the surface of the first coil 11 and the surface of the second coil 21, can be measured by using various elemental analyzers, e.g., a spectrophotometer such as a Raman spectrophotometer and a near-infrared spectrophotometer, an X-ray fluorescence spectrometer, or the like. The amount of the drug per coil unit length or the amount of the drug per coil unit area on each of the surface of the first coil 11 and the surface of the second coil 21 may be obtained by extracting the drug arranged on the coil with a solvent, and analyzing the concentration. A kind of the solvent is not particularly limited. However, for example, ethanol, methanol, acetone, ethyl acetate, acetonitrile, N,N-dimethylacetamide, propanol, chloroform, or benzyl alcohol can be used.

In the indwelling system 1, the drug 40 preferably adheres to the surface of the first coil 11. In the indwelling system 1, the drug 40 may adhere to the surface of the second coil 21 such that the amount of the drug per coil unit length is less than that on the first coil 11. A drug may not necessarily adhere to the surface of the second coil 21.

The kind of the drug 40 (drug substance) arranged on the first coil 11 and the kind of the drug 40 (drug substance) arranged on the second coil 21 may be the same or different from each other.

The amount of the drug arranged per unit length on the surface of the second coil 21 is preferably half or less of the amount of the drug arranged per unit length on the surface of the first coil 11, more preferably one third thereof or less, and even more preferably one fourth thereof or less. The amount of the drug arranged per unit length on the surface of the second coil 21 may be 1/1000 or more of the amount of the drug arranged per unit length on the surface of the first coil 11, may be 1/500 thereof or more, or may be 1/100 thereof or more. In a case where the amount of the drug is in such a range, even when the drug is applied to the coil, the coil maintains flexibility, so that the coil can be indwelt in an aneurysm while operability is ensured.

As shown in FIG. 2 to FIG. 3, the drug 40 is preferably arranged on the first coil 11 over the entirety of the first coil 11 in the longitudinal axis direction x. Thus, the amount of the drug arranged on the first coil 11 can be made large, so that the required therapeutic effect is likely to be achieved. The drug may be arranged on merely a part of the first coil 11 in the longitudinal axis direction x.

As shown in FIG. 2, the drug 40 is preferably arranged on the first coil 11 on the outer circumferential surface 112 and the inner circumferential surface 113 of the first coil 11. For example, the drug 40 preferably adheres to the outer circumferential surface 112 and the inner circumferential surface 113 of the first coil 11. Thus, the amount of the drug arranged on the first coil 11 can be made large, so that the required therapeutic effect is likely to be achieved.

As shown in FIG. 3, it is preferable that the drug 40 is arranged on the first coil 11 on the outer circumferential surface 112 of the first coil 11 and is not arranged on the inner circumferential surface 113 of the first coil 11. That is, the drug 40 is preferably arranged merely on the outer circumferential surface 112 of the first coil 11. For example, it is preferable that the drug 40 adheres to the outer circumferential surface 112 of the first coil 11, and does not adhere to the inner circumferential surface 113 of the first coil 11. The inner circumferential surface 113 of the first coil 11 is distant from the inner surface of an aneurysm as compared with the outer circumferential surface 112. Therefore, in general, the drug 40 arranged on the inner circumferential surface 113 is unlikely to be released early as compared with the drug arranged on the outer circumferential surface 112. Therefore, even when the drug 40 is not arranged on the inner circumferential surface 113 of the first coil 11, the required therapeutic effect can be achieved.

For the same reason as for the first coil 11, the drug 40 may be arranged on the second coil 21 as follows. The drug 40 may be arranged over the entirety of the second coil 21 in the longitudinal axis direction x. The drug 40 may be arranged on merely a part of the second coil 21 in the longitudinal axis direction x. As shown in FIG. 4, the drug 40 may be arranged on the outer circumferential surface 212 and the inner circumferential surface 213 of the second coil 21. For example, the drug 40 may adhere to the outer circumferential surface 212 and the inner circumferential surface 213 of the second coil 21. As shown in FIG. 5, the drug 40 may be arranged on the outer circumferential surface 212 of the second coil 21, and may not necessarily be arranged on the inner circumferential surface 213 of the second coil 21. For example, the drug 40 may adhere to the outer circumferential surface 212 of the second coil 21, and may not necessarily adhere to the inner circumferential surface 213 of the second coil 21.

As shown in FIG. 6, it is preferable that the drug is not arranged over the entirety of the surface of the second coil 21, and is not arranged over the entirety of the second coil 21.

In a case where the indwelling system 1 also has the third coil 31 as shown in FIG. 7, the drug 40 is preferably arranged on the surface of the third coil 31 as shown in FIG. 8. For example, the drug 40 preferably adheres to the surface of the third coil 31. In a case where a drug is arranged on the coil having such a hardness, the drug can be applied to an affected site at an amount required for treatment while influence of degradation of operability is reduced.

A kind of the drug 40 arranged on the third coil 31 and a kind of the drug 40 arranged on the first coil 11 may be the same or different from each other. A kind of the drug 40 arranged on the third coil 31 and a kind of the drug 40 arranged on the second coil 21 may be the same or different from each other.

The drug 40 is preferably arranged over the entirety of the third coil 31 in the longitudinal axis direction x. The drug 40 may be arranged on merely a part of the third coil 31 in the longitudinal axis direction x. As shown in FIG. 8, the drug 40 is preferably arranged on the outer circumferential surface 312 and the inner circumferential surface 313 of the third coil 31. For example, the drug 40 preferably adheres to the outer circumferential surface 312 and the inner circumferential surface 313 of the third coil 31. As shown in FIG. 9, it is preferable that the drug 40 is arranged on the outer circumferential surface 312 of the third coil 31 and is not arranged on the inner circumferential surface 313 of the third coil 31. That is, the drug 40 may be arranged merely on the outer circumferential surface 312 of the third coil 31. For example, the drug 40 may adhere to the outer circumferential surface 312 of the third coil 31 and may not necessarily adhere to the inner circumferential surface 313 of the third coil 31.

The drug is preferably arranged on the surface of the third coil 31 such that an amount of the drug per coil unit length is more than that on the first coil 11. That is, the amount of the drug arranged per coil unit length on the surface of the third coil 31 is preferably more than the amount of the drug arranged per coil unit length on the surface of the first coil 11. Even when the coil having such a hardness becomes harder due to application of the drug, since influence of degradation of operability is small, the amount of the drug can be made large.

The amount of the drug arranged per coil unit length on the surface of the first coil 11 is preferably half or less of the amount of the drug arranged per coil unit length on the surface of the third coil 31, more preferably one third thereof or less, and even more preferably one fourth thereof or less. The amount of the drug arranged per coil unit length on the surface of the first coil 11 may be 1/1000 or more of the amount of the drug arranged per coil unit length on the surface of the third coil 31, may be 1/500 thereof or more, or may be 1/100 thereof or more.

The amount of the drug (mg/mm²) per coil unit area on the surface of the third coil 31 may be more than the amount of the drug (mg/mm²) per coil unit area on the surface of the first coil 11.

When the first coil 11 is equally divided into two portions which are the distal portion and the proximal portion in the longitudinal axis direction x, the amount of the drug arranged per coil unit length on the distal portion of the first coil 11 may be more than the amount of the drug arranged per coil unit length on the proximal portion. When the second coil 21 is equally divided into two portions which are the distal portion and the proximal portion in the longitudinal axis direction x, the amount of the drug arranged per coil unit length on the distal portion of the second coil 21 may be more than the amount of the drug arranged per coil unit length on the proximal portion. When the third coil 31 is equally divided into two portions which are the distal portion and the proximal portion in the longitudinal axis direction x, the amount of the drug arranged per coil unit length on the distal portion of the third coil 31 may be more than the amount of the drug arranged per coil unit length on the proximal portion. Thus, the drug can be applied to an aneurysm at an amount required for treatment, and, furthermore, flexibility is enhanced at the proximal portion as compared with the distal portion and operability thus becomes good.

The amount of the drug arranged per coil unit length on the distal portion of the second coil 21 may be less than the amount of the drug arranged per coil unit length on the proximal portion of the first coil 11. The amount of the drug arranged per coil unit length on the distal portion of the second coil 21 may be less than the amount of the drug arranged per coil unit length on the proximal portion of the third coil 31. The amount of the drug arranged per coil unit length on the distal portion of the first coil 11 may be less than the amount of the drug arranged per coil unit length on the proximal portion of the third coil 31.

The amount of the drug arranged on the distal portion or the proximal portion in the coil may be measured by cutting the coil along the radial direction of the coil at a position at which the coil is equally divided into two portions in the longitudinal axis direction x and separating the coil into the distal portion and the proximal portion.

The kind of the drug is not particularly limited as long as the drug is required for prevention and treatment for an affected site. The drug may have at least one of an anti-inflammatory effect, an antioxidant effect, an antihypertensive effect, a vasoconstriction inhibitory effect, an anticoagulant effect, and a shear stress sensing inhibitory effect. At least one of the drug 40 arranged on the first coil 11 and the drug 40 arranged on the second coil 21 preferably has at least one of an anti-inflammatory effect, an antioxidant effect, an antihypertensive effect, a vasoconstriction inhibitory effect, an anticoagulant effect, and a shear stress sensing inhibitory effect. Examples of the drug include selective serotonin reuptake inhibitors (SSRI) (fluoxetine, sertraline, paroxetine, and the like), DPP-4 inhibitors (sitagliptin, linagliptin, alogliptin, and the like), HMG-CoA reductase inhibitors (atorvastatin, pitavastatin, rosuvastatin, pravastatin, simvastatin, fluvastatin, lovastatin, mevastatin, cerivastatin, and the like), non-steroidal anti-inflammatory drugs (NSAIDs) (ibuprofen, naproxen, celecoxib, and the like), angiotensin II receptor blockers (ARB) (losartan, valsartan, telmisartan, and the like), tocopherol acetate (vitamin E acetate, Eviprostat, Esterol, and the like), ascorbic acid (Asconale, Cinal, Cefirol), edaravone (Radicut, free radical scavenger, and the like), N-acetyl-L-cysteine (NAC), calcium antagonist (amlodipine, nifedipine, diltiazem, and the like), diuretics (furosemide, trichlormethiazide, spironolactone, and the like), angiotensin-converting enzyme inhibitors (ACE) (enalapril, lisinopril, perindopril, and the like), β-blockers (metoprolol, atenolol, bisoprolol, and the like), α-blockers (prazosin, terazosin, doxazosin, and the like), αβ-blockers (carvedilol, labetalol, butoxamine, and the like), nitrate (nitroglycerin, isosorbide dinitrate, and the like), prostacyclin analogs (epoprostenol, treprostinil, and the like), anticoagulants (heparin, heparin derivatives, warfarin, anti-thrombin agents such as dabigatran, rivaroxaban, and the like), and antiplatelet agents (aspirin, clopidogrel, ticagrelor, and the like).

In at least one of the first coil 11, the second coil 21, and the third coil 31, the drug 40 may adhere to the surface of the coil or may indirectly adhere to the surface of the coil via a bioadhesive. The kind of the material of the bioadhesive is not particularly limited. However, for example, polysaccharide adhesives such as collagen, chitosan, and gelatin, polyethylene glycol-based hydrogel adhesives, and protein adhesives such as fibrin and collagen, can be used.

The drug is preferably encapsulated in a capsule. The drug can be effectively released when the coil is delivered to an affected site in a living body. In at least one of the first coil 11, the second coil 21, and the third coil 31, the drug encapsulated in a capsule may adhere to the surface of the coil or may indirectly adhere to the surface of the coil via a bioadhesive.

The size of the capsule is preferably 10 nm or more, more preferably 50 nm or more, and even more preferably 100 nm or more, and preferably 500 nm or less, more preferably 400 nm or less, and even more preferably 200 nm or less.

The drug is preferably encapsulated in a capsule containing a biodegradable material. At least one of the drug 40 arranged on the first coil 11 and the drug 40 arranged on the second coil 21 is more preferably encapsulated in a capsule containing a biodegradable material. As the biodegradable material, a bioabsorbable polymer, a natural polymer, a decellularized biological tissue or cell, or a combination thereof can be used. As the bioabsorbable polymer, at least one of polylactic acid (PLA), poly-L-lactic acid (PLLA), polyglycolic acid (PGA), a copolymer of lactic acid and glycolic acid (PLGA), polycaprolactone (PCL), and polydioxanone (PDS) is preferably used. As the natural polymer, at least one of collagen, laminin, fibroin, gelatin, glycosaminoglycan, chitin, chitosan, hyaluronic acid, and polypeptide is preferably used. As the biodegradable material, in particular, PLGA is preferable.

The capsule preferably has a structure in which a plurality of filaments of the bioabsorbable polymer are condensed into a spherical shape. The number of the filaments of the bioabsorbable polymer is preferably 10,000 or more, more preferably 20,000 or more, and even more preferably 30,000 or more, and the number of the filaments of the bioabsorbable polymer may be 100,000 or less, 90,000 or less, or 80,000 or less. Among them, the capsule preferably has a structure in which 10,000 or more and 100,000 or less of the filaments of PLGA are condensed into a spherical shape.

The glass transition temperature of the material forming the capsule is not particularly limited, and may be, for example, 40°C or higher, 41°C or higher, or 42°C or higher, and is allowed to be 50°C or lower, 49°C or lower, or 48°C or lower.

The surface of the capsule is preferably covered with a coating material. The coating material can inhibit falling and elution of the drug into blood while the indwelling instrument is delivered into a living body. The material forming the coating material is preferably a water-soluble polymer from the viewpoint of preventing initial burst of the drug. Examples of the material of the coating material include carboxylmethylcellulose, hydroxypropylcellulose, methylcellulose, hydroxyethylcellulose, polyvinyl alcohol, alginic acid, pectin, gum arabic, gellan gum, guar gum, xanthan gum, carrageenan, and gelatin.

A surfactant may be applied on the surface of the capsule. In a case where the surfactant is applied, the cell membrane fluidity of the capsule is enhanced, and penetration of a drug into cell membranes can be enhanced. The kind of the surfactant is not particularly limited, but a nonionic surfactant is preferable, and the nonionic surfactant is, for example, polyoxyethylene sorbitan monolaurate, and is preferably Tween (registered trademark) 20, Tween (registered trademark) 80, or the like.

As shown in FIG. 2 to FIG. 3, the first coil 11 may have a first head portion 14 at the distal end portion. The first head portion 14 covers a part of the first wire 12 in order to prevent the distal end of the first wire 12 from coming into direct contact with an inner wall surface of a living body. The first head portion 14 may be in contact with the first stretch resistant member 15 or may not necessarily be in contact therewith.

As shown in FIG. 4 to FIG. 6, the second coil 21 may have a second head portion 24 at the distal end portion. The second head portion 24 covers a part of the second wire 22 in order to prevent the distal end of the second wire 22 from coming into direct contact with an inner wall surface of a living body. The second head portion 24 may be in contact with the second stretch resistant member 25 or may not necessarily be in contact therewith.

As shown in FIG. 8 to FIG. 9, the third coil 31 may have a third head portion 34 at the distal end portion. The third head portion 34 covers a part of the third wire 32 in order to prevent the distal end of the third wire 32 from coming into direct contact with an inner wall surface of a living body. The third head portion 34 may be in contact with the third stretch resistant member 35 or may not necessarily be in contact therewith.

Hereinafter, when a structure common to the first head portion 14 and the second head portion 24 (preferably, a structure common to the first head portion 14, the second head portion 24, and the third head portion 34) is described, these head portions or each head portion may be simply referred to as head portion.

Each of the shapes of the head portions is not particularly limited, and may be, for example, a hemispherical, a hemiellipsoidal, a columnar, or a polygonal-prism-like shape. The shapes of the first head portion 14, the second head portion 24, and the third head portion 34 may be the same or different from each other.

The head portion may be joined to at least one of the outer surface and the inner surface of the coil. A part of the head portion may be disposed in the lumen at the distal end portion of the coil in order to prevent the head portion from falling. The proximal end of the head portion may be disposed on the distal side as compared with the distal end of the wire, or the proximal end of the head portion may be disposed on the proximal side as compared with the distal end of the wire.

The head portion may be formed of a metal material or a resin. Examples of the resin forming the head portion include thermoplastic resins and ultraviolet curable resins. As the resin forming the head portion, ester-based resins such as epoxy acrylate-based resins, urethane acrylate-based resins, polyester acrylate-based resins, and polyethylene terephthalate-based resins, and olefin-based resins such as polypropylene can be used. As the metal forming the head portion, the metals described as examples for the wire can be used. The materials of the wire and the head portion may be the same or different from each other. The materials forming the first head portion 14 and the second head portion 24 may be the same or different from each other. The materials forming the first head portion 14, the second head portion 24, and the third head portion 34 may be the same or different from each other.

As can be understood from FIG. 1 to FIG. 3, the first in-vivo indwelling instrument 10 preferably includes the first coil 11, and the first stretch resistant member 15 disposed in the lumen 111 of the first coil 11. As can be understood from FIG. 1 and FIG. 4 to FIG. 6, the second in-vivo indwelling instrument 20 preferably includes the second coil 21, and the second stretch resistant member 25 disposed in the lumen 211 of the second coil 21. As can be understood from FIG. 7 to FIG. 9, the third in-vivo indwelling instrument 30 preferably includes the third coil 31, and the third stretch resistant member 35 disposed in the lumen 311 of the third coil 31. Hereinafter, when a structure common to the first stretch resistant member 15 and the second stretch resistant member 25, preferably, a structure common to the first stretch resistant member 15, the second stretch resistant member 25, and the third stretch resistant member 35, is described, these stretch resistant members or each stretch resistant member may be referred to as stretch resistant member.

The stretch resistant member inhibits the coil from stretching in the longitudinal axis direction x during operation. The stretch resistant member is preferably disposed in the lumen of the coil. The stretch resistant member may be a long member composed of a single wire or a stranded wire. The stretch resistant member has a longitudinal axis direction, and has a first end and a second end in the longitudinal axis direction. The stretch resistant member may be composed of a single layer or a plurality of layers in the radial direction perpendicular to the longitudinal axis direction. The stretch resistant member may have an inner layer composed of a stranded wire of a plurality of wires, and an outer layer including a resin composition disposed on the outer side of the inner layer. Merely one stretch resistant member or a plurality of the stretch resistant members may be disposed in the lumen of the coil.

The stretch resistant member may be formed of a resin or a metal. Examples of the resin forming the stretch resistant member include polyester resins such as polyethylene terephthalate, polyamide resins such as nylon, and polyolefin resins such as polyethylene and polypropylene. The stretch resistant member formed of the resin enhances flexibility and enhances delivery performance of the indwelling instrument. The stretch resistant member formed of the resin can avoid rupture due to metal fatigue at the time of delivery. By making the stretch resistant member longer than the coil, or using a stretchable material for the stretch resistant member, when the coil is disposed in an aneurysm, tension due to insufficient length of the stretch resistant member causing the end portion of the coil to linearly stretch can be reduced. Examples of the metal forming the stretch resistant member include platinum, gold, rhodium, palladium, rhenium, silver, nickel, titanium, tantalum, tungsten, and alloys thereof, and stainless steel.

The stretch resistant member may be formed of a material different from the material of the wire forming the coil. For example, the coil may be formed of a platinum-tungsten alloy, and the stretch resistant member may be formed of polypropylene resin.

The shape of the cross-section of the stretch resistant member, which is perpendicular to the longitudinal axis direction, may be a circular, an oblong, or a polygonal shape, or a combination thereof.

The outer diameter of the stretch resistant member is preferably less than half the inner diameter of the coil and more preferably one third thereof or less in order to easily dispose the stretch resistant member in the lumen of the coil. The outer diameter of the stretch resistant member is preferably one-fifteenth of the inner diameter of the coil or more and more preferably one-tenth thereof or more in order to prevent rupture of the stretch resistant member.

The stretch resistant member may have a linear, a wavy, or a helical shape, or a combination thereof.

The first end side of the stretch resistant member may be connected to the distal end portion of the coil, specifically, to the distal end portion of the wire forming the coil. The second end side of the stretch resistant member may be connected to the proximal end portion of the coil, specifically, to the proximal end portion of the wire forming the coil. The second end side of the stretch resistant member may be connected to a connection portion (described below) which connects the coil and a pusher to each other. The stretch resistant member may be disposed in the lumen of the coil in a state in which the stretch resistant member is folded back midway in the coil in the longitudinal axis direction x. In this case, it is preferable that the folded portion of the stretch resistant member is connected to the distal end portion or the proximal end portion of the coil, and the first end side and the second end side are connected to the proximal end portion or the distal end portion of the coil, or the distal end portion of the connection portion. In FIG. 2 to FIG. 3, the first stretch resistant member 15 has a folded portion 151 at which the first stretch resistant member 15 is folded back midway in the longitudinal axis direction, and the folded portion 151 is connected to the distal end portion of the first coil 11, and the first end side and the second end side are connected to a first connection portion 18 described below. Also in FIG. 4 to FIG. 6, similarly, a folded portion 251 of the second stretch resistant member 25 is connected to the distal end portion of the second coil 21, and the first end side and the second end side are connected to a second connection portion 28 described below. Also in FIG. 8 to FIG. 9, similarly, a folded portion 351 of the third stretch resistant member 35 is connected to the distal end portion of the third coil 31, and the first end side and the second end side are connected to a third connection portion 38 described below.

Examples of a method for connecting the stretch resistant member and another member to each other include fusion bonding, welding, crimping such as caulking, adhesion by an adhesive, physical fixing methods such as engagement, linking, binding, and ligation, and combinations thereof. In the description herein, the "connection" implies not only direct connection between two components, but also indirect connection between two components via one or more other components.

As shown in FIG. 10, the drug 40 may be arranged on the surface of the first stretch resistant member 15. Thus, an amount of the drug arranged in the first in-vivo indwelling instrument 10 including the first coil 11 can be made large. Therefore, the required therapeutic effect is easily achieved. Moreover, an effect of sustained release of the drug can also be enhanced. For example, a part or the entirety of the outer surface of the first stretch resistant member 15 can be coated with the drug 40. As shown in FIG. 11, a drug may not necessarily be arranged on the surface of the first stretch resistant member 15 or a drug may not necessarily be arranged on the entirety of the outer surface of the first stretch resistant member 15.

As shown in FIG. 12, the drug 40 may be arranged on the surface of the second stretch resistant member 25. Thus, an amount of the drug arranged in the second in-vivo indwelling instrument 20 including the second coil 21 can be made large. Therefore, the required therapeutic effect is easily achieved. Moreover, an effect of sustained release of the drug can also be enhanced. For example, a part or the entirety of the outer surface of the second stretch resistant member 25 can be coated with the drug 40. The drug may be arranged on the surface of the second stretch resistant member 25 such that an amount of the drug per unit length of the stretch resistant member is less than that on the first stretch resistant member 15. Alternatively, as shown in FIG. 13, preferably, no drug is arranged on the surface of the second stretch resistant member 25, and, more preferably, no drug is arranged on the entirety of the outer surface of the second stretch resistant member 25. Thus, the second stretch resistant member 25 can be prevented from being hardened due to application of the drug.

As shown in FIG. 14, the drug 40 is preferably arranged on the surface of the third stretch resistant member 35. Thus, an amount of the drug arranged in the third in-vivo indwelling instrument 30 including the third coil 31 can be made large. Therefore, the required therapeutic effect is easily achieved. Moreover, an effect of sustained release of the drug can also be enhanced. For example, a part or the entirety of the outer surface of the third stretch resistant member 35 can be coated with the drug 40. As shown in FIG. 15, a drug may not necessarily be arranged on the surface of the third stretch resistant member 35, and a drug may not necessarily be arranged on the entirety of the outer surface of the third stretch resistant member 35.

An amount of the drug arranged per unit length of the stretch resistant member on the surface of the third stretch resistant member 35 may be more than an amount of the drug arranged per unit length of the stretch resistant member on the surface of the first stretch resistant member 15. An amount of the drug arranged per unit length of the stretch resistant member on the surface of the third stretch resistant member 35 may be the same as an amount of the drug arranged per unit length of the stretch resistant member on the surface of the first stretch resistant member 15.

Similarly to the coil, in at least one of the first stretch resistant member 15, the second stretch resistant member 25, and the third stretch resistant member 35, the drug 40 may adhere to the surface of the stretch resistant member or may indirectly adhere to the surface of the stretch resistant member via a bioadhesive. Similarly to the coil, in at least one of the first stretch resistant member 15, the second stretch resistant member 25, and the third stretch resistant member 35, the drug 40 encapsulated in the capsule may adhere to the surface of the stretch resistant member or may indirectly adhere to the surface of the stretch resistant member via a bioadhesive. As a kind of the bioadhesive, the description for the kind of the bioadhesive adhered to the coil can be referred to. As the structure of the capsule, the description for the structure of the capsule adhered to the coil can be referred to.

As shown in FIG. 1, in the indwelling system 1, the first in-vivo indwelling instrument 10 may include the first coil 11, the first connection portion 18 connected to the proximal end portion of the first coil, and a first pusher 17 connected to the first coil 11 via the first connection portion 18. The second in-vivo indwelling instrument 20 may include the second coil 21, the second connection portion 28 connected to the proximal end portion of the second coil 21, and a second pusher 27 connected to the second coil 21 via the second connection portion 28. As shown in FIG. 7, in the indwelling system 1, the third in-vivo indwelling instrument 30 may include the third coil 31, the third connection portion 38 connected to the proximal end portion of the third coil 31, and a third pusher 37 connected to the third coil 31 via the third connection portion 38.

Hereinafter, when a structure common to the first pusher 17 and the second pusher 27 (preferably a structure common to the first pusher 17, the second pusher 27, and the third pusher 37) is described, these pushers or each pusher may be simply referred to as pusher. When a structure common to the first connection portion 18 and the second connection portion 28 (preferably a structure common to the first connection portion 18, the second connection portion 28, and the third connection portion 38) is described, these connection portions or each connection portion may be simply referred to as connection portion.

The in-vivo indwelling instrument preferably has a detachment mechanism for detaching the coil from the pusher. Examples of the detachment mechanism include hydraulic, electrical, and mechanical detachment mechanisms. Among them, an electrical detachment mechanism is preferably used. In the detachment mechanism, preferably, the connection portion is heated and cut by electrical or thermal energy supplied via the pusher, and the coil is thus detached from the pusher. In this case, preferably, the connection portion is heated by high-frequency electric current supplied between the coil and the counter electrode, and the coil is detached from the pusher.

The shape of the connection portion is not particularly limited, but the shape may be a wire-like, a bar-like, a columnar, a polygonal-prism-like, a cylindrical, a polygonal-tubelike, a truncated-cone-like, or a truncated-polygonal-pyramid-like shape, or a combination thereof.

The connection portion preferably includes a material which is dissolved or melted by heating. The connection portion can be melted by Joule heat. Such a material is, for example, a synthetic resin material, and it is preferable to use, for example, hydrophilic resins of synthetic polymeric substances such as polyvinyl alcohol-based polymers the examples of which include polyvinyl alcohol (PVA), PVA crosslinked polymers, PVA water-absorbing gel freezing/thawing elastomers, and ethylene-vinyl alcohol copolymers.

The pusher is a bar-like or a wire-like member used for holding the indwelling instrument and pushing the indwelling instrument toward the distal side. The pusher may be composed of a single member or a plurality of members. The pusher may be composed of a wire member, a coil member, or a combination thereof. The pusher may be formed of an electrically conductive material such as stainless steel.

### 2. Method for manufacturing in-vivo indwelling instrument

The method for manufacturing the in-vivo indwelling instrument according to one embodiment of the present invention includes applying the drug when the coil has a hardness of a first reference value or more, and applying no drug when the coil has a hardness of less than the first reference value. By including such a step, the drug is applied to the coil in which a rate of change in coil hardness between before and after application of the drug is small, and therefore, even when the coil becomes hard due to application of the drug, influence of degradation of operability of the coil can be reduced. The coil having such a configuration also allows the drug to be applied to an affected site at an amount required for treatment. Furthermore, the coil in which the coil hardness and an amount of the drug are well-balanced can be manufactured, so that appropriate coils can be obtained according to the Framing, Filling, and Finishing phases.

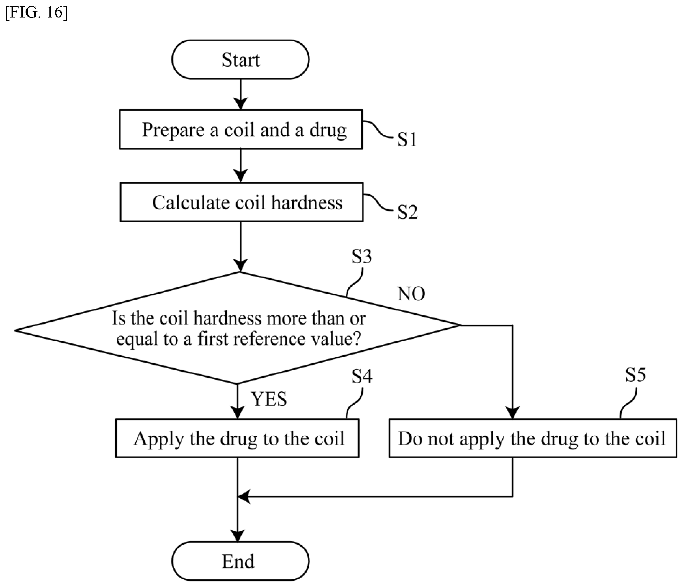

The method for manufacturing the in-vivo indwelling instrument according to one embodiment of the present invention will be described with reference to FIG. 16 to FIG. 17. FIG. 16 to FIG. 17 are flow charts showing the method for manufacturing the in-vivo indwelling instrument according to one embodiment of the present invention.

The first coil 11, the second coil 21, or the third coil 31 having the drug applied thereto can be obtained by performing the manufacturing method described herein.

Firstly, as shown in FIG. 16, preparing the coil and the drug is preferably performed (step S1). The coil preferably has a longitudinal axis direction, a circumferential direction, and a radial direction. The coil preferably has a distal end and a proximal end in the longitudinal axis direction. The proximal side of the coil refers to a side closer to a hand of a user or an operator with respect to the longitudinal axis direction of the coil, and the distal side refers to a side opposite to the proximal side, i.e., a side closer to a target to be treated. The radial direction of the coil refers to a coil radius direction. The inner direction in the radial direction of the coil refers to a direction toward the longitudinal axis center side of the coil. The outer direction in the radial direction refers to a direction radially extending from the longitudinal axis center toward the side opposite to the inner direction side. The circumferential direction of the coil refers to a direction around the longitudinal axis.

The coil has a structure in which one wire or a plurality of wires are helically wound. Examples of the wire include a single wire, a stranded wire, and a coil wire wound into a coil-like shape. Among them, a single wire is preferable. The wire is preferably other than a coil wire.

A coil formed by helically winding the wire may be referred to as primary coil. A coil formed by further bending and forming the primary coil into a helical shape or a three-dimensional shape may be referred to as secondary coil. The coil prepared in step S1 may be either the primary coil or the secondary coil.

For the details of the configuration of the coil prepared in step S1, the above description for the configuration of the first coil 11, the second coil 21, or the third coil 31 to which the drug has yet to be applied as described in "1. Indwelling system for in-vivo indwelling instrument" can be referred to.

After step S1, calculating a hardness of the prepared coil (step S2) may be performed. In step S2, the hardness S (unit: N/mm) of the prepared coil (primary coil) can be calculated according to the following equation. $S = {{D}_{1}}^{4} \times G / \left(8{{D}_{2}}^{3}\times n\right)$ in which D₁ represents the outer diameter (unit: mm) of the wire, G represents a shear modulus (unit: Pa(N/mm²)), D₂ represents the outer diameter (unit: mm) of the coil, and n represents the number of winding times in the coil (no unit). In a case where the outer diameter of the wire is not constant in the longitudinal axis direction, the outer diameter D₁ of the wire represents the average value of the outer diameters in the longitudinal axis direction of the wire. In a case where the outer diameter of the coil is not constant in the longitudinal axis direction, the outer diameter D₂ of the coil represents the average value of the outer diameters in the longitudinal axis direction of the coil. The number n of winding times represents the number of winding times as counted when the coil is viewed from the side at such an angle that the number of winding times becomes maximum. Step S2 may be omitted in a case where the coil hardness is known by using, for example, a product catalogue for the coil.

As shown in FIG. 16, determining whether or not the hardness of the coil is the first reference value or more (step S3) is preferably performed. In a case where the hardness of the coil is the first reference value or more, the drug is applied (step S4). In a case where the hardness of the coil is less than the first reference value, no drug is applied (step S5).

The determination in step S3 may be performed by a person or may be performed by using a processing device such as a CPU or a microprocessor.

In step S3 to step S5, the first reference value is preferably 5.0×10⁻⁹ N/mm or more and 1.5×10⁻⁸ N/mm or less. Even when the coil having such a hardness becomes hard due to application of the drug, influence of degradation of operability can be reduced. The coil having the hardness of the first reference value or more is preferably used for at least one of the Framing and Filling phases.

The first reference value is more preferably 8.0×10⁻⁹ N/mm or more and even more preferably 1.0×10⁻⁸ N/mm or more. The first reference value is preferably less than 3.5×10⁻⁸ N/mm, and is allowed to be 3.0×10⁻⁸ N/mm or less or 2.0×10⁻⁸ N/mm or less. The coil having such a hardness allows the drug to be applied to an affected site at an amount required for treatment while influence of degradation of operability is reduced, even when the drug is applied to the coil.

In step S4, the drug is preferably applied to the surface of the coil. In step S4, the drug may be applied to the outer circumferential surface and the inner circumferential surface of the coil. In step S4, the drug may be applied to the outer circumferential surface of the coil, and the drug may not necessarily be applied to the inner circumferential surface of the coil.

In step S4, an amount of the drug to be applied per coil unit length may be different between the outer circumferential surface and the inner circumferential surface of the coil. For example, in step S4, an amount of the drug to be applied per coil unit length to the inner circumferential surface of the coil may be less than that on the outer circumferential surface of the coil.

In step S4, the method for applying the drug to the coil is not particularly limited. Examples of the method for applying the drug include brush coating, roll coating, dip coating, and spray coating. In any of the methods, as the drug to be applied to the surface of the coil, a liquid drug or a solution in which, for example, microcapsules formed by encapsulating the drug are in a solvent, can be used.

After step S4 has ended, the drug may be held on the surface of the coil as a drug layer, or may be held on the surface of the coil in the form of the microcapsules or the like.

For the kind and the configuration of the drug to be applied to the coil in step S4 and the kind of the bioadhesive, the description in "1. Indwelling system for in-vivo indwelling instrument" can be referred to.

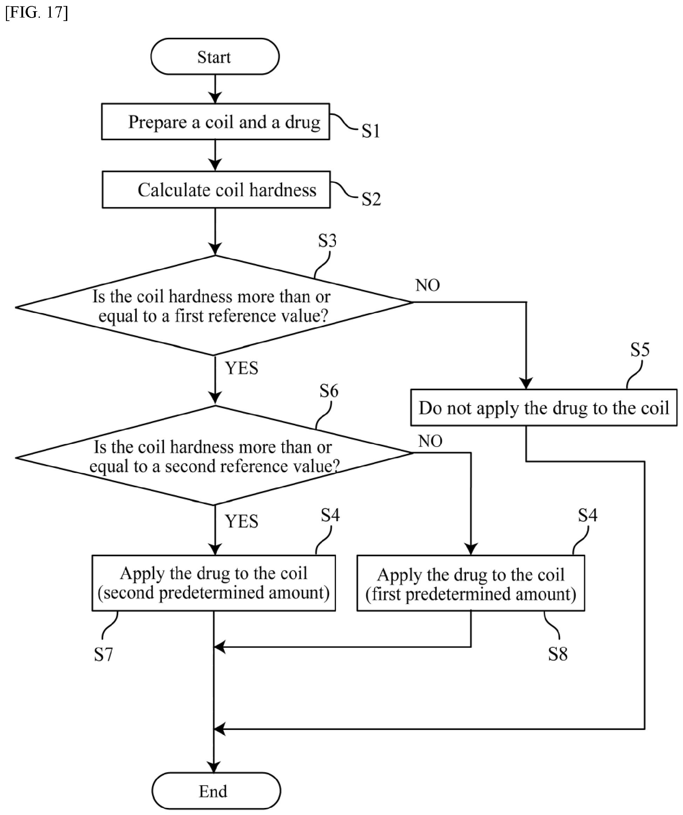

As can be understood from FIG. 17, whether or not the hardness of the coil is more than or equal to a second reference value more than the first reference value, may be further determined in step S3 or after step S3 (step S6). In applying the drug when the coil has a hardness of the first reference value or more and applying no drug when the coil has a hardness of less than the first reference value (step S3 to step S5), when the hardness of the coil is more than or equal to the second reference value more than the first reference value, an amount of the drug to be applied per coil unit length is preferably made more than that in a case where the hardness of the coil is the first reference value or more and less than the second reference value (step S7). Even when the coil having such a hardness becomes hard due to application of the drug, influence of degradation of operability can be reduced.

In the flow chart shown in FIG. 17, an amount of the drug (unit: mg/mm) which is applied per coil unit length to the coil in which the hardness of the coil is the first reference value or more and less than the second reference value is defined as a first predetermined amount, and an amount of the drug which is applied per coil unit length to the coil in which the hardness of the coil is more than or equal to the second reference value is defined as a second predetermined amount. That is, in steps S3 to S5, in a case where the hardness of the coil is more than or equal to the second reference value (YES in step S6), the drug is preferably applied to the coil at the second predetermined amount (step S7). In steps S3 to S5, in a case where the hardness of the coil is the first reference value or more and less than the second reference value (NO in step S6), the drug is preferably applied to the coil at the first predetermined amount (step S8). The second predetermined amount>the first predetermined amount>0 is satisfied.

In steps S7 to S8, the first predetermined amount is preferably half or less of the second predetermined amount, more preferably one third thereof or less, and even more preferably one fourth thereof or less. The first predetermined amount may be 1/1000 or more of the second predetermined amount, 1/500 thereof or more, or 1/100 thereof or more.

In at least one of step S7 and step S8, the drug is preferably applied to the surface of the coil. In at least one of step S7 and step S8, the drug may be applied to the outer circumferential surface and the inner circumferential surface of the coil. In at least one of step S7 and step S8, the drug may be applied to the outer circumferential surface of the coil, and the drug may not necessarily be applied to the inner circumferential surface of the coil.

In at least one of step S7 and step S8, an amount of the drug to be applied per coil unit length may be different between the outer circumferential surface and the inner circumferential surface of the coil. For example, in at least one of step S7 and step S8, an amount of the drug to be applied per coil unit length to the inner circumferential surface of the coil is preferably less than that on the outer circumferential surface of the coil.

The second reference value is preferably 3.5×10⁻⁸ N/mm or more and 4.5×10⁻⁸ N/mm or less. Even when the coil having such a hardness becomes hard due to application of the drug, influence of degradation of operability can be reduced. The coil having a hardness of the second reference value or more can be preferably used for at least one of the Framing and Filling phases, and can be more preferably used for the Framing phase.

The second reference value may be 4.0×10⁻⁸ N/mm or more or 8.0×10⁻⁸ N/mm or more, and is allowed to be 3.0×10⁻⁷ N/mm or less or 1.0×10⁻⁷ N/mm or less. The coil having such a hardness allows the drug to be applied to an affected site at an amount required for treatment while influence of degradation of operability is reduced, even when the drug is applied to the coil.

The present application claims priority based on Japanese Patent Application No. 2023-208509 filed on December 11, 2023. All the contents described in Japanese Patent Application No. 2023-208509 filed on December 11, 2023 are incorporated herein by reference.

### DESCRIPTION OF REFERENCE SIGNS

1: Indwelling system for in-vivo indwelling instrument
10: First in-vivo indwelling instrument
11: First coil
111: Lumen
112: Outer circumferential surface
113: Inner circumferential surface
12: First wire
14: First head portion
15: First stretch resistant member
17: First pusher
18: First connection portion
20: Second in-vivo indwelling instrument
21: Second coil
211: Lumen
212: Outer circumferential surface
213: Inner circumferential surface
22: Second wire
24: Second head portion
25: Second stretch resistant member
27: Second pusher
28: Second connection portion
30: Third in-vivo indwelling instrument
31: Third coil
311: Lumen
312: Outer circumferential surface
313: Inner circumferential surface
32: Third wire
34: Third head portion
35: Third stretch resistant member
37: Third pusher
38: Third connection portion
40: Drug
x: Longitudinal axis direction

## Claims

1. An indwelling system for an in-vivo indwelling instrument, comprising:
a first coil; and
a second coil having a coil hardness less than a hardness of the first coil, wherein
a drug is arranged on a surface of the first coil, and
a drug is arranged on a surface of the second coil such that an amount of the drug per coil unit length on the second coil is less than that on the first coil, or no drug is arranged on the surface of the second coil.

2. The indwelling system for the in-vivo indwelling instrument according to claim 1, wherein the drug is arranged on the first coil over an entirety of the first coil in a longitudinal axis direction.

3. The indwelling system for the in-vivo indwelling instrument according to claim 1 or 2, wherein the drug is arranged on the first coil on an outer circumferential surface and an inner circumferential surface of the first coil.

4. The indwelling system for the in-vivo indwelling instrument according to claim 1 or 2, wherein the drug is arranged on the first coil on an outer circumferential surface of the first coil and is not arranged on an inner circumferential surface of the first coil.

5. The indwelling system for the in-vivo indwelling instrument according to claim 1 or 2, further comprising a first stretch resistant member disposed in a lumen of the first coil, wherein
a drug is arranged on a surface of the first stretch resistant member.

6. The indwelling system for the in-vivo indwelling instrument according to claim 1 or 2, wherein the first coil has a coil hardness of 5.0×10⁻⁹ N/mm or more.

7. The indwelling system for the in-vivo indwelling instrument according to claim 1 or 2, further comprising a third coil having a coil hardness more than the hardness of the first coil, wherein
a drug is arranged on a surface of the third coil such that an amount of the drug per coil unit length on the third coil is more than that on the first coil.

8. The indwelling system for the in-vivo indwelling instrument according to claim 7, wherein the third coil has a coil hardness of 3.5×10⁻⁸ N/mm or more.

9. The indwelling system for the in-vivo indwelling instrument according to claim 1 or 2, wherein at least one of the drug arranged on the first coil and the drug arranged on the second coil is encapsulated in a capsule containing a biodegradable material.

10. The indwelling system for the in-vivo indwelling instrument according to claim 1 or 2, wherein at least one of the drug arranged on the first coil and the drug arranged on the second coil has at least one of an anti-inflammatory effect, an antioxidant effect, an antihypertensive effect, a vasoconstriction inhibitory effect, an anticoagulant effect, and a shear stress sensing inhibitory effect.

11. A method for manufacturing an in-vivo indwelling instrument, the method comprising applying a drug when a coil has a hardness of a first reference value or more, and applying no drug when the coil has a hardness of less than the first reference value.

12. The method for manufacturing the in-vivo indwelling instrument according to claim 11, wherein the first reference value is 5.0×10⁻⁹ N/mm or more and 1.5×10⁻⁸ N/mm or less.

13. The method for manufacturing the in-vivo indwelling instrument according to claim 11 or 12, wherein, in applying the drug when the coil has a hardness of the first reference value or more and applying no drug when the coil has a hardness of less than the first reference value, when the hardness of the coil is more than or equal to a second reference value more than the first reference value, an amount of the drug to be applied per coil unit length is made more than that in a case where the hardness of the coil is the first reference value or more and less than the second reference value.

14. The method for manufacturing the in-vivo indwelling instrument according to claim 13, wherein the second reference value is 3.5×10⁻⁸ N/mm or more and 4.5×10⁻⁸ N/mm or less.
